# EUROPEAN PATENT APPLICATION

(11) **EP 3 545 943 A1**
(43) Date of publication of application: **02.10.2019**
(21) Application number: 18164684.5
(22) Date of filing: 28.03.2018
(51) Int. Cl.: A61K 8/34, A61K 8/92, A61Q 5/02, A61Q 5/12, A61Q 11/00, A61Q 19/00, C11D 11/00, G01N 11/00, G01N 29/02

(54) **PROCESS FOR MAKING A LIQUID TREATMENT COMPOSITION**

(71) Applicant: The Procter & Gamble Company, Cincinnati, OH 45202 (US)
(72) Inventor: COROMINAS, Francesc, 1853 Strombeek-Bever (BE)
(74) Representative: P&G Patent Belgium UK

(57) **Abstract**

The present invention relates to a process for making a liquid treatment composition wherein the process comprises a step of passing the liquid treatment composition via a Rheology sensor.

## Description

### FIELD OF THE INVENTION

The present invention relates to a process for making a liquid treatment composition wherein the process comprises a step of passing the liquid treatment composition via a Rheology sensor.

### BACKGROUND OF THE INVENTION

Manufacture of liquid detergent compositions requires careful balance of ingredients and process steps. During manufacture aspects of the process may need to be adjusted, for example to add more of a particular ingredient or adjust the pH of the composition, to ensure it meets product specifications. If the final product does not meet preferred specifications, this could negatively impact the cleaning or care ability of the composition and so negatively affect the consumer experience. Further it could negatively impact factors such as physical stability or physical state of the product or regulatory compliance. Again, these can negatively affect the consumer experience.

Traditionally, during the manufacturing process samples are collected and analyzed to ensure the product meets specification. If it does not then it can be adjusted by addition of relevant components or ingredients, or the product is recycled back to make adjustments, or in some cases the product needs to be scrapped which is expensive and inefficient. This is inefficient as by the time the error has been noticed, the product has already moved down the production line and needs to be recycled back. This affects process throughput. Similarly, traditional methods do not allow early trend identification and hence fast interception to prevent product that does not meet specifications. All of this can be resource intensive both from a cost and 'manpower' perspective.

One characteristic that requires careful monitoring is composition rheology. Traditionally this has been achieved by taking samples and running single value viscosity measurements or making inline single value viscosity measurements.

However, treatment compositions are complex fluids with a non-Newtonian behavior. This means that there is not a single viscosity value that characterizes the liquid rheology, but rather a curve of viscosity versus shear rate is needed to characterize the liquid properly.

Therefore, there is a need in the art for an improved process to monitor rheology and preferably adjust in real time, characteristics of a treatment composition during manufacture.

It was surprisingly found that the process of the present invention overcame this technical challenge.

### SUMMARY OF THE INVENTION

A first aspect of the present invention is a process for making a liquid treatment composition comprising the steps of;
a. Mixing two or more ingredients together in a first mixing operation to create a liquid treatment composition;
b. Flowing the liquid treatment composition through a pipe, via a rheology sensor, wherein the rheology sensor, and wherein the rheology sensor periodically measures the liquid treatment composition and electronically transmits the data to a suitable electronic medium;
c. Interpreting the data to determine the rheology profile of the liquid treatment composition;
d. Comparing the rheology profile of the liquid treatment composition to predetermined acceptable rheology profile ranges;
e. If the rheology profile is outside of the acceptable rheology profile ranges adjusting one or more characteristics of the first mixing operation in order to bring the rheology profile of the liquid treatment composition back into acceptable ranges;
f. Flowing the liquid treatment composition to where optionally one or more further process steps occur;
g. Collecting the liquid treatment composition.

### BRIEF DESCRIPTION OF THE DRAWINGS

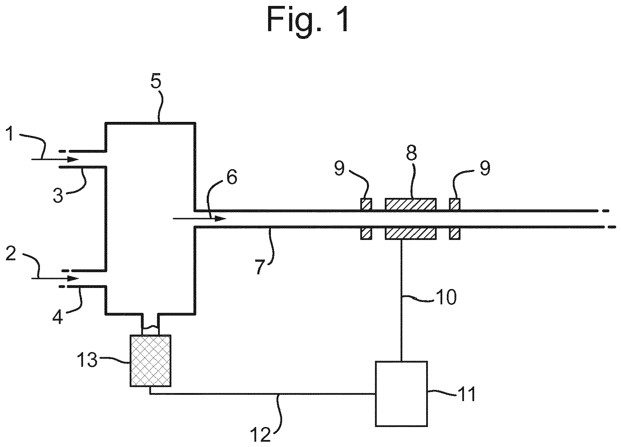

FIG. 1 discloses a process for making a liquid treatment composition comprising a rheology sensor, wherein the rheology sensor is not in physical contact with the liquid treatment composition.

### DETAILED DESCRIPTION OF THE INVENTION

### Process

The present invention relates to a process for making a liquid treatment composition. In relation to the liquid treatment composition of the present invention, the term 'liquid' encompasses forms such as dispersions, gels, pastes and the like. A dispersion for example is a liquid comprising solid or particulate matter contained therein.

The liquid treatment composition may also include gases in suitably subdivided form. However, the liquid treatment composition excludes forms which are non-liquid overall, such as tablets or granules.

The term 'treatment composition' includes any composition capable of wetting or treating a surface, wherein the surface could be flexible, e.g. a fabric, or hard, e.g. a floor or kitchen unit. The treatment composition may comprise a fabric laundry composition, a fabric softening composition, a hard surface cleaner, a hand dishwashing liquid, an automatic dishwashing composition or a combination thereof.

The liquid treatment composition is preferably selected from laundry detergents, hard surface cleaners, dish washing detergents or mixtures thereof, preferably wherein the liquid treatment composition is a liquid laundry detergent composition. The liquid treatment composition is described in more detail below.

The liquid treatment composition may be used as a final product sold to consumers or may be an intermediate composition which is further processed to make a final product for sale to consumers.

Without wishing to be bound by theory, the present invention provides a rheology profile of the liquid treatment composition. Traditionally, only single point viscosity measurements have been achieved which do not provide the same degree of composition information as a rheology profile which is a curve of viscosity versus shear rate. In addition the present invention provides the added benefit of in-line real time rheology profile measurements as opposed to offline measurements. This means the operator can react quickly to changes in the rheology profile in real time reducing wasted product that is not within specified criteria and/or product recycling needs. Furthermore, the present invention allows an automated system to adjust and bring the rheology profile back into acceptable ranges.

**Step a:** The process comprises a step a of mixing two or more ingredients together in a first mixing operation to create a liquid treatment composition. The liquid treatment composition is described in more detail below. Those skilled in the art will be aware of suitable ingredients. Suitable ingredients may be selected from water, non-aqueous solvents, surfactants, polymers, enzymes, dyes, perfumes or mixtures thereof.

Any suitable mixing operation may be used. Those skilled in the art will be aware of ways to mix the at least two or more ingredients. Mixing can be achieved via batch or continuous process. The two or more ingredients may be mixed together in a dynamic mixing vessel, preferably a paddle mixing vessel. The two or more ingredients may be mixed via a static mixer.

**Step b:** The process comprises a step b of flowing the liquid treatment composition through a pipe, via a rheology sensor. The rheology sensor may be in physical contact with the liquid treatment composition or may not be in physical contact with the liquid treatment composition. Without wishing to be bound by theory, by being in physical contact with the liquid treatment composition, the accuracy of the rheology sensor may be increased. When not in physical contact with the liquid treatment composition, this has the advantage of being a less intrusive measurement method. This allows for easier maintenance of the rheology sensor and simplifies cleaning of the pipe following a particular production run as cleaning of the rheology sensor is not required.

The rheology sensor may be positioned on the outside of the pipe, on the inside of the pipe, within the wall of the pipe or a mixture thereof. If the rheology sensor is in physical contact with the liquid treatment composition then preferably the rheology sensor is located on the inside of the pipe, within the wall of the pipe or a mixture thereof. If the rheology sensor is not in physical contact with the rheology sensor then preferably the rheology sensor is located on the outside of the pipe, within the wall of the pipe or a mixture thereof.

The rheology sensor periodically measures the liquid treatment composition and electronically transmits the data to a suitable electronic medium. The liquid treatment composition is periodically scanned. A single scan of the liquid treatment composition as it flows via the rheology sensor can be achieved within 0.4 seconds. Periodic scans can be performed on any preferred frequency. Preferably, the liquid treatment composition is scanned between every 0.4 and 120 seconds, more preferably between every 1 and 60 seconds.

The rheology sensor may be positioned in any suitable point along the pipe. Those skilled in the art will recognise suitable positions. The rheology sensor may be positioned along a section of pipe comprising no inlet valves or mixing elements. Alternatively, the rheology sensor may be positioned along a section of pipe comprising a mixing element wherein the mixing element is described as above. The rheology sensor is described in more detail below.

Preferably, the liquid treatment composition also flows via a pressure sensor, a temperature sensor of a mixture thereof. The pressure sensor, the temperature sensor or both may be positioned before the rheology sensor, after the rheology sensor or both. The pressure sensor may be positioned before the rheology sensor, after the rheology sensor or both. The temperature sensor may be positioned before the rheology sensor, after the rheology sensor or both. Without wishing to be bound by theory, pressure and temperature can affect rheology measurement. Therefore, a more accurate rheology profile can be calculated by adjusting for temperature and pressure differences or fluctuations.

The pipe is used to flow the liquid treatment composition through the manufacturing apparatus to a collection point. The pipe can be of any suitable material or construction and those skilled in the art will recognise suitable structures and materials. The pipe has to be suitable for the flow of the liquid treatment composition, and be of sufficient size to achieve a flow of liquid treatment composition to achieve desired manufacturing throughput. It is also preferred that the diameter and internal volume of the pipe is sufficient to minimise blockages of the liquid treatment composition and will be dependent on the chemical make-up of the liquid treatment composition, e.g. viscosity, presence of particulates etc. Those skilled in the art will be aware of relevant pipe diameters and volumes dependent on chemical nature of the liquid treatment composition.

The pipe may be constructed from any suitable material. Those skilled in the art will be aware of suitable materials. The pipe may be constructed from metal, glass, plastic or a mixture thereof. Suitable metals include stainless steel, carbon steel or a mixture thereof. Stainless steel may be selected from commercially available grades 316, 316L, 304, 304L or a mixture thereof. Carbon steel may be selected from commercially available CS-1018, Carpenter 20-CB3, 2205, AL6XN, Hastelloy C-276 or mixtures thereof. Preferably the pipe is constructed of stainless steel. Suitable plastics include, polypropylene, chlorinated polyvinylchloride, Teflon TFE, Fiber Reinforced Plastic (FRP) or a mixture thereof. Suitable FRP includes Derakane 411M.

It is also preferable that the pipe is constructed such that addition of other materials and/or process steps can be achieved to the liquid detergent composition as it is flowing through said pipe. For example, it is preferable that the pipe comprises one or more inlet valves within the wall of the pipe to allow addition of further ingredients commonly used in detergent compositions. It may be desirable to add certain ingredients up or down stream of the addition of other ingredients, for reasons of compatibility or mixing requirements.

The pipe may comprise one or more mixing elements. Those skilled in the art will be aware of suitable mixing elements. The mixing element may be selected from static mixers, dynamic mixers or a mixture thereof. The mixing element may be in the form of a small mixing tank such as a continuous stirred reactor.

**Step c:** The process comprises a step c of interpreting the data to determine the rheology profile of the liquid treatment composition. The data obtained from scanning the liquid treatment composition is interpreted in order to determine the Rheology profile of the liquid treatment composition.

Preferably the interpretation of the data is done via an automated system, most preferably via computer software. The computer software will utilize an algorithm in order to determine the rheology profile. Those skilled in the art will be aware of relevant computer software and algorithms that can be used to achieve this.

**Step d:** The process comprises a step d of comparing the rheology profile of the liquid treatment composition to pre-determined acceptable rheology profile ranges. Without wishing to be bound by theory, the 'acceptable ranges' are pre-determined by the manufacturer based on known rheology profiles in control compositions. If the rheology profile is outside of the range this can be alerted as an action in order to correct the deviation.

Preferably, the comparison is done via an automated system, most preferably via computer software. The computer software will use an algorithm to determine whether the rheology profile is within or outside of acceptable ranges. When outside of the acceptable ranges, the software can alert the manufacturer, by for example displaying a warning on an apparatus control screen. The software may also indicate what action needs to be taken to correct the issue and bring the rheology profile back within acceptable ranges.

**Step e:** The process comprises a step e of if the rheology profile is outside of the acceptable rheology profile ranges adjusting one or more characteristics of the first mixing operation in order to bring the rheology profile of the liquid treatment composition back into acceptable ranges.

Those skilled in the art will be aware of relevant corrective actions. Alternatively, the software of step d may enact an automatic corrective action, in other words, if the rheology profile is outside of acceptable ranges, the software will automatically instruct the manufacturing apparatus to take the necessary action to adjust this either with or without an alert to the manufacturer.

Adjustments may comprise addition of one or more ingredients, increase in levels of one or more ingredients, reduction in levels of one more ingredient, change to mixing speed, change to mixing temperature, change in mixing energy or a mixture thereof.

Preferably, the adjusting of one or more characteristics of the first mixing operation occurs within 180 seconds, preferably within 60 seconds, more preferably within 30 seconds of the rheology profile comparison in step d.

**Step f:** The process comprises a step f of flowing the liquid treatment composition to where optionally one or more further process steps occur. Those skilled in the art will be aware of relevant further process steps. Such steps could include addition of further ingredients, mixing, heating of the composition, cooling of the composition, or a mixture thereof. The addition of further ingredients in step f preferably includes acidic or alkali materials to adjust pH, viscosity adjustment agents, aesthetic dyes, perfumes, preservatives or a mixture thereof. Mixing steps could include passing the liquid detergent composition through a low or high shear static mixer, a low or high shear dynamic mixer or a mixture thereof.

Further process steps may comprise;
a. Flowing the liquid treatment composition via a buffer tank;
b. Flowing a portion of the liquid treatment composition via a feedback loop to the first mixing operation in step a;
c. a mixture thereof.

**Step g:** The process comprises a step g of collecting the liquid treatment composition. The collected liquid treatment composition may be used as a fully formulated consumer product or may serve as an intermediate product. If it is an intermediate product, then one or more further ingredients and/or process steps may be applied to the liquid detergent composition in order to result in a fully formulated consumer product.

Without wishing to be bound by theory, a further benefit of the present invention is that from a product quality standpoint, product may be 'released for sale' quicker, even in real time. Often manufactured product needs to be checked to ensure it meets certain manufacturing quality standards (e.g. levels of ingredients within pre-determined ranges) before it is permitted by the manufacturer to be sold to consumers. Often times such verification is done once the product is made. This means there is an additional step of storing the product ahead of it being released. This is time and cost ineffective. Since the process of the present invention achieves this during manufacture, product can be released quicker or even 'real time', i.e. at the point the manufacturing steps have been completed.

The liquid treatment composition may be collected into any suitable container. Those skilled in the art will be aware of appropriate containers. The liquid detergent composition may be collected in a bottle, a tub, a water-soluble unit dose article, a holding tank, sachets or a mixture thereof. Suitable containers are described in more detail below.

The process may be a continuous process, a batch process or a semi-continuous process, preferably the process is a continuous process.

A batch process is a process whereby a pre-determined volume of product is manufactured in an isolated system. Once a particular batch is made, it is removed from the system and a new batch is then started. This means there is a period of time between batches whereby no product is being manufactured. In a continuous process, materials are added constantly and product is manufactured and collected continuously. Unlike batch processes there is no interruption between when product is being manufactured.

A semi-continuous process is more where the process can be interrupted, for example have an intermediate storage stage but wherein the overall process per se is continuous.

Preferably, the liquid detergent composition is flowing at rate of between 100kg per hour and 150,000 kg per hour.

### Liquid treatment composition

The liquid treatment composition comprises two or more, preferably three or more ingredients. In other words, the treatment composition does not comprise a single ingredient, rather it comprises at least two different ingredients. Suitable ingredients are described in more detail below.

In the context of the present invention, a liquid treatment composition is a composition whereby at least two ingredients are present in liquid form. The ingredient may provide a cleaning benefit, a shine benefit, a softness benefit, a freshness benefit, a preferred viscosity, a preferred pH, a preferred color or a mixture thereof. Water may be an ingredient. However, the liquid treatment composition should not be understood to be made solely from one ingredient.

The liquid treatment composition may be selected from household care compositions, personal care compositions, oral care compositions or a mixture thereof

Household care compositions preferably comprise laundry detergents, fabric enhancers, hard surface cleaners, air care refreshers, dish washing detergents or mixtures thereof.

Personal care compositions preferably comprise skin care compositions, hair shampoos, hair conditions or a mixture thereof.

Oral care compositions preferably comprise toothpastes, mouth wash or a mixture thereof.

The liquid treatment composition may be used as a fully formulated consumer product or may serve as an intermediate product. If it is an intermediate product, then one or more further ingredients and/or process steps may be applied to the liquid detergent composition was collected in step g of the present process in order to result in a fully formulated consumer product.

The detergent ingredients may be mixed together in any suitable order in order to make the liquid treatment composition. The ingredients may all be mixed together and then flowed through the pipe or some of the ingredients may be initially mixed together and then flowed through the pipe after which further ingredient or ingredients are added whilst the liquid detergent composition is in transit through the pipe. Those skilled in the art will know appropriate order of mixing.

### Liquid treatment composition ingredients

The ingredient may be selected from any ingredient commonly used in a liquid treatment composition. Those skilled in the art will be aware of suitable ingredients.

The ingredient may be selected from surfactants, builders, chelants, polymers, non-aqueous solvents, dyes, perfumes, preservatives, antibacterial agents, viscosity modifiers, pH adjustment agents, enzymes, water or a mixture thereof.

Preferably, the ingredient may be selected from surfactants, builders, polymers, non-aqueous solvents, enzymes, water or a mixture thereof.

### Container

The liquid treatment composition may be collected in step g in a bottle, a tub, a water-soluble unit dose article, a holding tank, a sachet or a mixture thereof.

The liquid treatment composition may be collected in a bottle. Preferably, the liquid treatment composition is flowed via a nozzle into the bottle, after which the bottle is closed. Those skilled in the art will be aware of suitable nozzles to achieve this. The bottle may be of any shape and volume. Those skilled in the art will be aware of suitable shapes and volumes for the bottle.

The liquid treatment composition may be collected in a water-soluble unit dose article. Preferably, a first water-soluble film is deformed in a mould to form an open cavity. The liquid treatment composition is then added to the open cavity preferably via a nozzle. A second water-soluble film is then used to close the open cavity containing the liquid treatment composition, and the first and second films are then sealed together preferably via heat, solvent, pressure or a mixture thereof.

The water-soluble unit dose article comprises at least one water-soluble film shaped such that the unit-dose article comprises at least one internal compartment surrounded by the water-soluble film. The at least one compartment comprises the liquid treatment composition. The water-soluble film is sealed such that the liquid treatment composition does not leak out of the compartment during storage. However, upon addition of the water-soluble unit dose article to water, the water-soluble film dissolves and releases the contents of the internal compartment into the wash liquor.

The unit dose article may comprise more than one superposed orientation, i.e. one positioned on top of the other. Alternatively, the compartments may be positioned in a side-by-side orientation, i.e. one orientated next to the other.

Preferred film materials are preferably polymeric materials. The film material can, for example, be obtained by casting, blow-moulding, extrusion or blown extrusion of the polymeric material, as known in the art.
compartment, even at least two compartments, or even at least three compartments. The compartments may be arranged in
Preferred polymers, copolymers or derivatives thereof suitable for use as pouch material are selected from polyvinyl alcohols, polyvinyl pyrrolidone, polyalkylene oxides, acrylamide, acrylic acid, cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides including starch and gelatine, natural gums such as xanthum and carragum.

The liquid treatment composition may be collected in a holding vessel. The holding vessel may be used to temporarily or permanently store the liquid detergent composition. The liquid treatment composition may be temporarily stored in the holding vessel ahead of it being further processed or packaged into a relevant container. The holding vessel can be made from any suitable material and can have suitable dimension. Those skilled in the art will know suitable materials and dimensions.

### Rheology sensor

Preferably, the rheology sensors utilise ultrasound wave propagation through the liquid. Preferably, the ultrasound wave is generated using piezo-electric transducers comprised within the sensor. Preferably, the ultrasound wave is converted into voltage with piezo-electric transducers. Preferably, said piezo-electric transducers are located on the outer part of the pipe, so as to not be invasive into the liquid treatment composition. More preferably, the Rheology sensor comprises at least one piezo-electric transducer located on the outer surface of the pipe which measures ultrasound characteristics of the liquid treatment composition in the pipe and wherein in step c, a computer algorithm is used to interpret said ultrasound characteristics into a rheology profile of the liquid treatment composition.

Without wishing to be bound by theory, piezo electric transducers convert voltage into an ultrasound pressure wave and vice versa. This data can then be collected and interpreted with reference to standards to calculate the rheology profile of the liquid treatment composition. Every sensor emits ultrasound waves that travel through the liquid and the resulting ultrasound wave is received by another sensor. The propagation of the ultrasound wave is influenced by the liquid properties.

In order to get a complete analysis of the liquid properties inside the pipe, preferably an array of piezo-electric transducers are installed on the outer part of the pipe. Preferably, the pipe comprises between 2 and 20 piezo-electric transducers.

As an alternative to using piezo-electric transducers, a rheology sensor can also be based on any other suitable device or transducer that can generate ultrasound waves.

As a further alternative to using ultrasound waves, a rheology sensor can also be based on vibrations, oscillations, translational, rotational movements or combinations thereof, transmitted to and from any solid part that is in contact with the liquid treatment composition. The solid part can be the same pipe where the liquid is flowing through. A solid part can be any part or piece inserted inside the pipe where the liquid treatment composition is flowing through.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

### EXAMPLES

FIG. 1 discloses a process for making a liquid treatment composition comprising a rheology sensor. In more detail, the process comprises the addition of at least a first ingredient (1) via a first pipe (3) and a second ingredient (2) via second pipe (4) into a mixing zone (5) to create a liquid treatment composition (6). The liquid treatment composition (6) is flowed through a pipe (7) via a rheology sensor (8) wherein the rheology sensor is on the inside of the pipe, on the outside of the pipe or a mixture thereof. Preferably the rheology sensor (8) comprises a piezo-electric transducer. The liquid treatment composition (6) may be flowed via one or more further sensors, such as pressure or temperature sensors (9). The rheology sensor (8) periodically measures the liquid treatment composition (6) and electronically transmits the data via a first wire (10) to a suitable electronic medium (11). The data is interpreted via a suitable algorithm to determine the rheology profile of the liquid treatment composition (6) and this profile compared to a pre-determined acceptable rheology profile. If the rheology profile is outside of the acceptable rheology profile, the electronic medium (11) transmits instructions via a second wire (12) to a suitable means (13) to adjust one or more characteristics in the mixing zone (5) to bring the liquid treatment composition (6) back into acceptable ranges.

### Example 1

An apparatus was set up according to FIG. 1.

The Rheology sensor was supplied by Nederlandse Organisatie voor toegepastnatuurwetenschappelijk onderzoek (TNO).

A differential pressure sensor Optibar DP7060C was provided by Krohne.

An algorithm to interpret the data generated and compare to predetermined standards was provided by Universita Delgi Studi Di Cagliari (UNICA).

A basic liquid treatment composition comprising 87wt% of a base composition, 10wt% 1,2-propanediol, and 3wt% hydrogenated castor oil was prepared in the mixing zone (5) and the rheology measured by taking a sample manually.

The base composition comprises a mixture of surfactants, non-aqueous solvents, monoethanolamine, Glycerine, builders, chelants, polymers, salts and pH adjustment agents.

As an example of the accuracy achieved by the inline rheology sensor, the table 1 below details the results obtained from the sensor as compared to the rheology curve obtained from an off-line measurement carried out in a sample of liquid taken during the experiments. The off-line measurement was carried out in the lab using a rheometer obtained from DHR, TA Instruments. The data from the sensor i.e. "in-line rheology" is the data coming from the rheology sensor, after processing and interpreting it with the appropriate algorithm. The flow rate of the liquid composition was 2600 kg/h. As it can be seen in the table 1 below, the agreement between in-line and off-line rheology data is very good, with a difference of one to the other of less than 3%.

**Table 1**

| | In-line sensor | Off-line lab |
|---|---|---|
| Shear rate [1/s] | Viscosity [Pa·s] | Viscosity [Pa·s] |
| 0.1 | 5.40 | 5.60 |
| 1 | 1.95 | 1.91 |
| 10 | 0.89 | 0.87 |
| 100 | 0.60 | 0.61 |
| 1000 | 0.55 | 0.54 |

In addition a control algorithm to adjust one or more characteristics in the mixing zone (5) was also provided by UNICA. The liquid treatment composition was then flowed via the rheology sensor at a rate of 2600kg/h. Based on data acquired from the rheology sensor, the level of non-aquous solvent and hydrogenated castor oil in the mixing zone (5) were adjusted three times in order to reach the target viscosity.

The rhology profile as measure by the rheology sensor after each adjustment was recorded and 3 different shear rate values shown. Results can be seen in Table 2

**Table 2**

| Shear rate (1/s) | Viscosity (Pa.s) | | | | |
|---|---|---|---|---|---|
| | Start | Adjustment 1 | Adjustment 2 | Adjustment 3 | Target |
| 0.1 | 4.68 | 5.88 | 6.78 | 7.01 | 7.54 |
| 1 | 1.80 | 2.10 | 2.30 | 2.35 | 2.52 |
| 10 | 0.87 | 0.92 | 0.93 | 0.94 | 0.98 |

As can be seen from Table 2, the process of the present invention allowed inline measurement and same time adjustment of the rheology profile of the liquid treatment composition to bring it into target profile ranges.

## Claims

1. A process for making a liquid treatment composition comprising the steps of;
a. Mixing two or more ingredients together in a first mixing operation to create a liquid treatment composition;
b. Flowing the liquid treatment composition through a pipe, via a rheology sensor, wherein the rheology sensor periodically measures the liquid treatment composition and electronically transmits the data to a suitable electronic medium;
c. Interpreting the data to determine the rheology profile of the liquid treatment composition;
d. Comparing the rheology profile of the liquid treatment composition to predetermined acceptable rheology profile ranges;
e. If the rheology profile is outside of the acceptable rheology profile ranges adjusting one or more characteristics of the first mixing operation in order to bring the rheology profile of the liquid treatment composition back into acceptable ranges;
f. Flowing the liquid treatment composition to where optionally one or more further process steps occur;
g. Collecting the liquid treatment composition.

2. The process according to claim 1 wherein said process is a continuous process, a batch process or a semi-continuous process, preferably wherein the process is a continuous process.

3. The process according to any preceding claims, wherein in step f the further process step may comprise;
a. Flowing the liquid treatment composition via a buffer tank;
b. Flowing a portion of the liquid treatment composition via a feedback loop to the first mixing operation in step a;
c. a mixture thereof.

4. The process according to any preceding claims wherein the liquid detergent composition is flowing at rate of between 100kg per hour and 150,000 kg per hour.

5. The process according to any preceding claims wherein the Rheology sensor is in physical contact with the liquid treatment composition or wherein the Rheology sensor is not in physical contact with the liquid treatment composition, and wherein the Rheology sensor is located on the outside of the pipe, on the inside of the pipe, within the wall of the pipe of a mixture thereof.

6. The process according to any preceding claims wherein the Rheology sensor comprises at least one piezo-electric transducer which measures ultrasound propagation characteristics of the liquid treatment composition in the pipe and wherein in step c, a computer algorithm is used to interpret said ultrasound characteristics into a rheology profile of the liquid treatment composition., or the rheology sensor detects rheology based on vibrations, oscillations, translational, rotational movements or combinations thereof, transmitted to and from any solid part that is in contact with the liquid treatment composition, or a mixture thereof.

7. The process according to any preceding claims wherein in step d the comparison is enabled by computer software.

8. The process according to any preceding claims wherein in step e the adjusting one or more characteristics of the first mixing operation occurs within 180 seconds, preferably within 60 seconds, more preferably within 30 seconds of the rheology profile comparison in step d.

9. The process according to any preceding claims wherein adjusted characteristics in step e comprise, addition of one or more ingredients, increase in levels of one or more ingredients, reduction in levels of one more ingredient, change to mixing speed, change to mixing temperature, change of mixing energy or a mixture thereof.

10. The process according to any preceding claims wherein in step b, the liquid treatment composition is flowed via a temperature sensor, a pressure sensor or a combination thereof, and wherein the temperature sensor, the pressure sensor or a combination thereof is positioned before the rheology sensor, after the rheology sensor or both.

11. The process according to any preceding claims wherein the liquid treatment composition is collected in step g in a bottle, a tub, a water-soluble unit dose article, a holding tank or a mixture thereof.

12. The process according to any preceding claims wherein the liquid treatment composition is selected from household care compositions, personal care compositions, oral care compositions or a mixture thereof

13. The process according to claim 11, wherein household care compositions comprise laundry detergents, fabric enhancers, hard surface cleaners, air care refreshers, dish washing detergents or mixtures thereof.

14. The process according to claim 11 wherein personal care compositions comprise skin care compositions, hair shampoos, hair conditions or a mixture thereof.

15. The process according to claim 11 wherein oral care compositions comprise toothpastes, mouth wash or a mixture thereof.
